# EUROPÄISCHE PATENTSCHRIFT

(11) **EP 1 946 740 B1**
(45) Veröffentlichungstag und Bekanntmachung des Hinweises auf die Patenterteilung: **29.06.2011**
(21) Anmeldenummer: 07024029.6
(22) Anmeldetag: 12.12.2007
(51) Int. Cl.: A61K 6/10, C08L 83/04, C08L 71/02

(54) **Zweikomponenten-Abformmassen**
Two component impression material
Masses de moulage à deux composants

(30) Priorität: 18.01.2007 DE 102007003604
(43) Veröffentlichungstag der Anmeldung: 23.07.2008
(73) Patentinhaber: Heraeus Kulzer GmbH, 63450 Hanau (DE)
(72) Erfinder: Grundler, Andreas, 41542 Dormagen (DE)
(74) Vertreter: Kühn, Hans-Christian

(56) Entgegenhaltungen:
- EP-A- 1 290 998
- DE-A1- 19 719 438

## Beschreibung

Die Erfindung betrifft Zweikomponenten-Abformmassen.

Bekannt ist in der Dentaltechnik die Kombination aus einem kondensationsvernetzenden und einem additionsvernetzendem Silikon zum Zwecke einer zweistufigen Kinetik, die Vorteile im Handling ergeben soll. Ein derartiges Produkt ist Panasil ® binetics, der Firma Kettenbach, Eschenburg, DE. Die entsprechenden Schutzrechte und Anmeldungen W0200258641A1, DE10103446 B4 und DE20121446 U1 sind auf zweistufig härtbare Silikonmaterialien gerichtet, die Vinylgruppen zusammen mit Si-OH-Gruppen und/oder Alkinylgruppen enthalten, sowie jeweils die entsprechenden Katalysatoren für die Kondensations- und die Additionsvernetzung.

DE4439769 A1 beschreibt Kunststoffe mit mindestens einem Alkoxysilyl-, Ether- und Urethangruppen enthaltenden Polyadditionsprodukt mit einer überwiegend linearen Molekülstruktur mit aliphatisch oder cycloaliphatisch gebundenen Ether- und Urethansegmenten, die mindestens einen Katalysator für die Kondensation der Silangruppen enthalten.

Gemäß DE3928987 A1 wird der Restmonomergehalt selbsthärtenden Prothesenmaterials durch zusätzliche Lichtpolymerisation vermindert.

In DE19847635 A1 werden härtbare polymerisierbarer Dentalmassen mit Organosiloxanpartikeln als Zusatz beschrieben. Als Dentalmasse können Mischungen von Alginat- und Agar-Agar-Abformmassen und Polyether- und Silikonmassen eingesetzt werden.

Kondensationsvernetzende Polyether, wie das Heraeus Produkt P2 (beschrieben z.B. in EP269819 A2), weisen im Vergleich zu additionsvernetzenden Silikonen (z.B. Heraeus Produkt Flexitime) und kondensationsvernetzenden Silikonen (z.B. dem Heraeus Produkt Optosil) eine systemimmanent gesteigerte *Oberflächenklebrigkeit* auf.

EP 1290998 beschreibt Dentalabdruckmaterialien enthaltend additionsvernetzende Silikone, zu denen zur Erhöhung der Hydrophilie additionsvernetzende Polyether in der Anwesenheit von nicht-ionischen grenzflächenaktiven Mitteln und/oder Polyethermodifizierten Silikonöl zugegeben werden.

Bedingt durch den säurekatalysierten Abbindemechanismus des Polyethers ist zudem eine *Inhibition durch Feuchtigkeits- oder Wassereinfluss* systembedingt nicht ganz auszuschließen.

Andererseits weisen Polyether durch ihre Ether-Kettenstruktur eine natürliche Hydrophilie auf, die den Vorgang des Anfließens an feuchte Oberflächen wirkungsvoll unterstützt und auch während des gesamten Abbindevorganges jener von klassischen A- und C-Silikonen überlegen ist.

Auf Zusätze von Detergentien/Tensiden kann bei den Polyethern daher in den meisten Fällen verzichtet werden.

Aufgaben der Erfindung sind gegenüber dem diskutierten Stand der Technik die folgenden:
- Reduktion der Klebrigkeit von kondensationsvernetzenden Polyethern,
- Verringerung der Inhibition der Abbindereaktion von kondensationsvernetzenden Polyethern durch Feuchtigkeit und wässrige Lösungen wie Speichel,
- Erhöhung der Reißdehnung von kondensationsvernetzenden Polyethern.

Diese Aufgaben werden durch Zusammensetzungen gemäß Anspruch 1 und den weiteren Ansprüchen gelöst.

Die Erfindung betrifft somit Zweikomponenten-Abformmassen enthaltend
- **I** als Basispaste eine Mischung aus:
   **A** funktionellen Polyethern mit linearer oder verzweigter Hauptkette und
   **B** mindestens einem Organo-hydrogen-Polysiloxan-Vernetzer (B).
- **II** als Katalysatorpaste eine Mischung aus:
   **C** organischen und/oder anorganischen Säuren,
   **D** mindestens einem vinyl-terminierten Organopolysiloxan (D) und
   **E** 10 - 1000 ppm, basierend auf der Summe von (B) und (D), einer silikonlöslichen Edelmetall-Katalysator-Komponente.

Dabei stellen insbesondere Komponente
**A** alkoxysilylfunktionelle Polyether mit linearer oder verzweigter Hauptkette und Komponente
**C** Wasser und organische und/oder anorganische Säuren in einem Gewichtsverhältnis von 1:0,01 bis 1:40
dar.

In einer bevorzugten Ausführungsform weist Komponente
**A** ein mittleres Molekulargewicht (Mn) von 200 bis 20000, einen Gehalt an Polyethergruppen von 20 - 95%, einen Gehalt an Alkoxysilylgruppen -SR1 R2R3 von 0,2 bis 25%
wobei R1, R2, R3 unabhängig voneinander H, Alkyl oder Alkoxy bedeuten, auf.

Komponente **A** kann zusätzlich einen Gehalt an Urethangruppen von 0-10% oder einen Gehalt an Harnstoffgruppen von 0 - 10% aufweisen.

Als weitere Komponenten können 5-70% für Polyether- und/oder für A-Silikon-Abformmaterialien übliche Verdünner und/oder 10 bis 90% für Polyether- und/oder für A-Silikon-Abformmaterialien übliche Füllstoffe und/oder 0 bis 20% für Polyether- und/oder für A-Silikon-Abformmaterialien übliche Additive und/oder 0 - 2% für A-Silikone übliche Wasserstoff Fänger enthalten sein.

Der silikonlösliche Edelmetall-Katalysator enthält vorzugsweise Platin.

Zusätzlich können 0,01 bis 7,5 % eines ionischen und/oder nichtionischen Tensides und /oder polyethermodifizierten Silicon Öls und/oder linearen und/oder verzweigten Polyethers zur weiteren Erhöhung der Hydrophilie enthalten sein.

Dabei ist der Polyether bevorzugt vom Typ Polypropylenoxid oder Polyethylenoxid.

Die Erfindung betrifft auch die Verwendung der beschriebenen Komponenten A bis E zur Herstellung von klebfreiem Abformmaterial für medizinische, zahnärztliche und zahntechnische Zwecke.

Die Mischverhältnisse zwischen Polyether und A-Silikon-Basispasten, die Mischverhältnisse zwischen Polyether und A-Silikon-Katalysatorpasten und die Mischungsverhältnisse zwischen den neuartig zusammengesetzten Katalysator und Basispasten sind nicht erfindungswesentlich limitiert und können in weiten Grenzen variieren.

Es können erfindungsgemäß Mischungen von Materialien einer Reihe von Viskositäten eingesetzt werden, darunter auch solche mit sogenannten "light"-Viskositätsbereichen.

Mit den Zusammensetzungen der Erfindungen werden die vorteilhafte stabile Abbindekinetik (der Silane) unter feuchten Bedingungen, die geringe Klebrigkeit (der Silane), eine gute Oberflächenbenetzbarkeit (der Polyether) und ein gutes Anfließverhalten (der Polyether) kombiniert.

In einer besonderen Ausführungsform werden dazu Rezepturen bekannter A-Silikone mit den Rezepturen bekannter kondensationsvernetzender Polyether kombiniert.

Die Basispasten der besonderen Ausführungsform enthalten die Bestandteile der Basispasten bekannter A-Silikone und bekannter kondensationsvernetzender Polyether, die Katalysatorpasten die Bestandteile der Katalysatorpasten bekannter A-Silikone und bekannter kondensationsvernetzender Polyether. Die neuartigen Basis- und Katalysatorpasten der besonderen Ausführungsform werden in herkömmlicher Weise vermischt und dann zum Abformen verwendet.

Als vorteilhaft hat sich für die Katalysatorpasten ein Mengenverhältnis von ca. 3 Gew.-Teilen Polyetherbasispaste und ca. 5 Gew.-Teilen A-Sitikonbasispaste erwiesen. Für die Basispasten ist ein Mengenverhältnis von ca. 1:1 1 vorteilhaft.

Es ist aber auch möglich, eigens besondere, davon abweichende Rezepturen zu formulieren.

Es ist weiterhin möglich, eine Kombination von additionsvernetzenden Silikonen und anderen Polyethern, wie z.B. den Aziridinopolyethern (z.B. Impregum von 3M ESPE) vorzunehmen.

Das folgende Beispiel erläutert die Erfindung näher (%-Angaben beziehen sich auf das Gewicht):

### Beispiel

### I Anmischung einer Katalysatorpaste aus

### I 1 3,3 g einer Kat-Paste des kondensationsvernetzenden Polyethers P2 light

[enthaltend ca. 33% eines Poly(propylenoxid)-diols (MW 2000), ca. 59% Quarzmehl, ca. 4% pyrogene Kieselsäure, ca. 2% einer ca. 15% igen wässrigen Lösung von p-Toluolsulfonsäurehydrat und ca. 2% eines Stabilisators] und

### I 2 5 g einer Basispaste des A-Silikons Flexitime Correct Flow enthaltend ca. 30% eines vinyl-terminierten Polysiloxans, ca. 20% eines Organohydropolysiloxanes, ca. 40% Füllstoff und 5% Hilfsstoffe zu einer gemischten Basispaste.

### II Anmischung einer Basispaste aus

**II** 1 6,6 g kondensationsvernetzenden Polyethers P2 light enthaltend ca. 23% einer Mischung alkoxysilylfunktioneller Polyether mit linearer Hauptkette mit einem mittleren Molekulargewicht von 800 bis 20000, mit einem Gehalt an Polyethergruppen von 20 - 90% und einem Gehalt an Alkoxysiliylgruppen von 0,2 - 25% und einem Gehalt an Urethangruppen von 0 - 10%, enthaltend ca. 34% Verdünner, enthaltend ca. 42 % Quarzmehl und ca. 1 % Stabilisator.

**II 2** 5 g einer Katalysatorpaste des A-Silikons Flexitime Correct Flow enthaltend ca. 60% vinylterminierter Polysiloxane, ca. 35% Füllstoffe, ca. 4 % Hilfsstoffe und < 1 % Platin Katalysator zu einer gemischten Katalysatorpaste

**III** Die gemischten Katalysator- und Basispasten werden im Verhältnis 1:1 1 vereinigt und für 20 sec. von Hand auf einem Anmischblock homogen vermischt. Man erhält eine Anmischung, die mit einer Verarbeitungszeit von ca. 3 min. und einer Abbindezeit von 6 min. zu einem gemischten Silikon/Polyether-Block abbindet.

### Vergleich mit Polyethermaterial P2:

Das Material weist überraschenderweise im Vergleich zur reinen Mischung aus den Polyether P2 Basis- und Katalysatorpasten keine Oberflächenklebrigkeit auf, und das Abbinden wird auch durch direkte Wassereinwirkung nicht beeinträchtigt. Dies lässt sich durch den folgenden Test belegen:

Wird die Anmischung C nach dem Homogenisieren auf einem Grindometer¹ ausgestrichen und nach 1 min. für einen Zeitraum von 4 min. in ein auf 37°C temperiertes Wasserbad gelegt, so weist die Oberfläche im Gegensatz zu einer P2 Anmischung keine Inhibitionsschicht und keine Klebrigkeit auf.
¹Ein Grindometer ist ein Gerät zum Messen der Körnigkeit eines Pigments.

Zur Prüfung wird eine Lack- bzw. Mahlgutprobe mit dem Rakel auf der keilförmigen Vertiefung des Grindometers verteilt. Dann wird mit dem Auge anhand der Skala*diejenige* Schichtdicke bestimmt, bei der der Lackfilm infolge auftretender streifen- oder punktförmigen Spuren rauh aussieht. Messung des Pigment-Ausreibgrades mit dem Grindometer (eng. to grind = mahlen) nach *Hegman,* beschrieben in DIN 53 203 bzw. DIN EN 21 524 und ISO 1524.

Zudem weist die Mischung im Vergleich zu einer Mischung aus dem reinen A-Silikon Basis und den Katalysatorpasten eine gesteigerte Oberflächenhydrophilie auf.

### Viskosimetrie

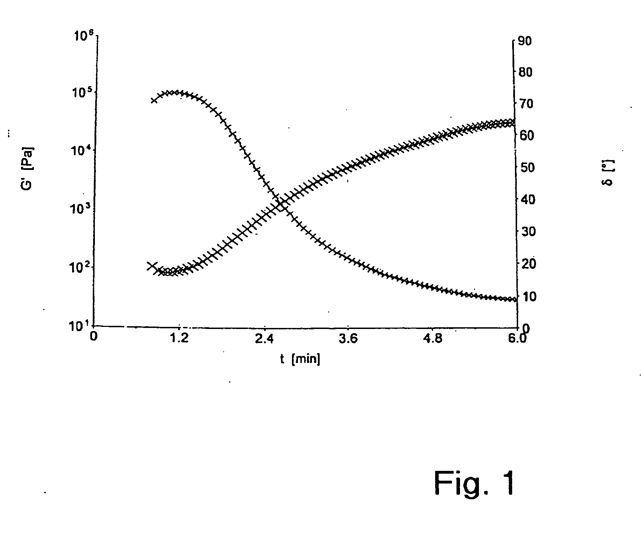

Die Überprüfung der Abbindekinetik mittels Viskosimetrie² zeigt, dass das Material C keine zweistufige Reaktionskinetik aufweist, sondern dass A-Silikon und Polyether synchron polymerisieren. Das Diagramm der Fig. 1 zeigt den entsprechenden zeitabhängigen Viskositätsverlauf.
² Methode zur Bestimmung der Verarbeitungszeit und Abbindezeit von Reaktivsystemen. Reaktivsysteme (z.B. Abformmassen) werden durch Polymerisation und/ oder Vernetzung von einem viskosen in einen festen, elastischen Zustand überführt. Bei einer oszillierenden Beanspruchung kann durch die Messung viskoelastischer Kenngrößen (Speichermodul, Verlustmodul bzw. Verlustwinkel) dieser Phasenübergang kontinuierlich verfolgt werden. Dazu wird in einem Rheometer mit Oszillationsmodus die zu messende Masse bei einer Temperatur von 30°C zwischen zwei Platten (Abstand 0,5 mm) oszillierend bei einer Frequenz von 1 Hz deformiert und der Deformationswinkel kontinuierlich gemessen. Ausgewertet wird die Deformationsamplitude als Speichermodul G' und der Phasenwinkel (Verlustwinkel) zwischen der Deformationskraft und der Auslenkung der Platten, delta. Der Verlustwinkel ändert sich normalerweise ausgehend von großen Winkeln, max. 90° (rein viskos), zu kleinen Winkeln, Minimum 0° (rein elastisch).

Das Ende der Verarbeitungszeit wird hier als der Zeitpunkt von Mischbeginn an definiert, an dem die abbindende Masse gleich große Anteile an viskosen und elastischen Eigenschaften besitzt (delta = 45°). Als Abbindezeit wird die Zeit von Mischbeginn bis zum Zeitpunkt bei dem die Masse sich schon weitgehend elastisch verhält (delta = 10°) definiert.

## Patentansprüche

1. Zweikomponenten-Abformmasse enthaltend
• **I** als Basispaste eine Mischung aus:
• **A** funktionellen Polyethern mit linearer oder verzweigter Hauptkette und
• **B** mindestens einem Organo-hydrogen-Polysiloxan-Vemetzer (B)
• **II** als Katalysatorpaste eine Mischung aus
• **C** organischen und/oder anorganischen Säuren,
• **D** mindestens einem vinyl-terminierten Organopolysiloxan (D) und
• **E** 10 - 1000 ppm, basierend auf der Summe von (B) und (D), einer silikonlöslichen Edelmetall-Katalysator-Komponente.

2. Abformmasse nach Anspruch 1, wobei Komponente
• A alkoxysilylfunktionelle Polyether mit linearer oder verzweigter Hauptkette und Komponente
• C Wasser und organische/und oder anorganische Säuren in einem Gewichtsverhältnis von 1:0,01 bis 1:40
darstellen.

3. Abformmasse gemäß Anspruch 2, wobei Komponente
• **A** ein mittleres Molekulargewicht (Mn) von 200 bis 20000, einen Gehalt an Polyethergruppen von 20 - 95%, einen Gehalt an Alkoxysilylgruppen -SR1 R2R3 von 0,2 bis 25% wobei R1, R2, R3 unabhängig voneinander H, Alkyl oder Alkoxy bedeuten, aufweist, und optional
Komponente A zusätzlich einen Gehalt an Urethangruppen von 0 - 10% oder einem Gehalt an Harnstoffgruppen von 0 - 10% aufweist.

4. Abformmasse nach einem der vorstehenden Ansprüche, **dadurch gekennzeichnet, dass** als weitere Komponenten
5-70 Gew.% für Polyether- und/oder A-Silikon-Abformmaterialien übliche Verdünner und/oder
10 bis 90 Gew.% für Polyether- und/oder A-Silikon-Abformmaterialien übliche Füllstoffe und/oder
0 bis 20 Gew.% für Polyether- und/oder A-Silikon-Abformmaterialien übliche Additive und/oder 0-2 Gew.% für A-Silikone übliche Wasserstoff Fänger enthalten sind.

5. Abformmasse nach einem der vorstehenden Ansprüche, wobei der silikonlösliche Edelmetall-Katalysator Platin enthält.

6. Abformmasse nach einem der vorstehenden Ansprüche enthaltend zusätzlich 0,01 bis 7,5 Gew.% eines ionischen und/oder nichtionischen Tensides und /oder polyethermodifizierten Silicon Öls und/oder linearen und/oder verzweigten Polyethers zur zusätzlichen Erhöhung der Hydrophilie.

7. Abformmasse nach Anspruch 7, wobei der Polyether vom Typ Polypropylenoxid oder Polyethylenoxid ist.

8. Verwendung von in Anspruch 1 beschriebenen Komponenten A bis E zur Herstellung von klebfreiem Abformmaterial für medizinische, zahnärztliche und zahntechnische Zwecke.

## Claims

1. Two-component elastic impression compound containing
• **I** as base paste, a mixture of:
• **A** functional polyethers with a linear or branched main chain and
• **B** at least one organo-hydrogen-polysiloxane cross-linker (B)
• **II** as catalyst paste, a mixture of
• **C** organic and/or inorganic acids,
• **D** at least one vinyl-terminated organopolysiloxane (D), and
• **E** 10 - 1,000 ppm, based on the sum of (B) and (D), of a silicone-soluble noble metal catalyst component.

2. Elastic impression compound according to claim 1, whereby component
• A is alkoxysilyl-functional polyethers with a linear or branched main chain, and component
• C is water and organic and/or inorganic acids at a weight ratio of 1:0.01 to 1:40.

3. Elastic impression compound according to claim 2, whereby component
• **A** has a mean molecular weight (Mn) from 200 to 20,000, a content of polyether groups of from 20 - 95%, a content of alkoxysilyl groups -SR1 R2R3 of from 0.2 to 25%, whereby R1, R2, R3, independent of each other, are H, alkyl or alkoxy, and optionally component A comprises, in addition, a content of urethane groups of from 0 - 10% or a content of urea groups of from 0 - 10%.

4. Elastic impression compound according to any one of the preceding claims, **characterised in that** it contains as further components
5 - 70 % by weight common diluting agents for polyether and/or A-silicone elastic impression materials and/or 10 to 90 % by weight common filling agents for polyether and/or A-silicone elastic impression materials and/or
0 to 20 % by weight common additives for polyether and/or A-silicone elastic impression materials and/or 0 - 2 % by weight common hydrogen getter for A-silicone elastic impression materials.

5. Elastic impression compound according to any one of the preceding claims, whereby the silicon-soluble noble metal catalyst contains platinum.

6. Elastic impression compound according to any one of the preceding claims, containing, in addition, 0.01 to 7.5 % by weight of an ionic and/or non-ionic tenside and/or polyether-modified silicon oil and/or linear and/or branched polyether to additionally increase the hydrophilicity.

7. Elastic impression compound according to claim 6, whereby the polyether is of the polypropylene oxide type or polyethylene oxide type.

8. Use of components A through E described in claim 1 for the manufacture of tack-free elastic impression material for purposes of medicine, dentistry, and dental technology.

## Revendications

1. Masse de moulage bicomposant contenant
• **I** comme pâte de base un mélange de :
• **A** polyéthers fonctionnels avec une chaîne principale linéaire ou ramifiée et
• **B** au moins un agent de réticulation à base d'organo-hydrogène-polysiloxane (B)
• Il en tant que pâte de catalyseur un mélange de
• **C** acides organiques et/ou inorganiques,
• **D** au moins un organopolysiloxane à terminaison vinylique (D) et
• **E** 10 à 1000 ppm, en se basant sur la somme de (B) et (D), d'un composant de catalyseur en métal noble soluble dans la silicone.

2. Masse de moulage selon la revendication 1, dans laquelle le composant
• **A** représente un polyéther à fonction alcoxysilyle avec une chaîne principale linéaire ou ramifiée et le composant
• **C** représente de l'eau et des acides organiques et/ou inorganiques dans un rapport pondéral de 1/0,01 à 1/40.

3. Masse de moulage selon la revendication 2, dans laquelle le composant
• **A** présente un poids moléculaire moyen (Mn) de 200 à 20000, une teneur en groupes polyéther de 20 à 95 %, une teneur en groupes alcoxysilyle -SR1 R2R3 de 0,2 à 25 %, R1, R2, R3 signifiant indépendamment les uns des autres H, alkyle ou alcoxy, et en option le composant A présente en outre une teneur en groupes uréthane de 0 à 10 % ou une teneur en groupes urée de 0 à 10 %.

4. Masse de moulage selon l'une quelconque des revendications précédentes, **caractérisée en ce qu'**en tant que composants supplémentaires
5 à 70 % en poids de diluants usuels pour des matériaux de moulage en polyéther et/ou silicone A et/ou 10 à 90 % en poids de charges usuelles pour des matériaux de moulage en polyéther et/ou silicone A et/ou
0 à 20 % en poids d'additifs usuels pour des matériaux de moulage en polyéther et/ou silicone A et/ou 0 à 2 % en poids de capteurs d'hydrogène usuels pour des matériaux de moulage en silicone A sont contenus.

5. Masse de moulage selon l'une quelconque des revendications précédentes, dans laquelle le catalyseur en métal noble soluble dans la silicone contient du platine.

6. Masse de moulage selon l'une quelconque des revendications précédentes, contenant en outre 0,01 à 7,5 % en poids d'un tensioactif ionique et/ou non ionique et/ou d'une huile de silicone modifiée avec un polyéther et/ou d'un polyéther linéaire et/ou ramifié pour l'augmentation supplémentaire de l'hydrophilie.

7. Masse de moulage selon la revendication 6, dans laquelle le polyéther est du type oxyde de polypropylène ou oxyde de polyéthylène.

8. Utilisation de composants A à E décrits à la revendication 1 pour la fabrication d'un matériau de moulage non adhésif à des fins médicales, dentaires et de technique dentaire.
